(19) Europäisches Patentamt
European Patent Office
Office européen des brevets

(11) Veröffentlichungsnummer: **0 075 110**
**A2**

# (12) EUROPÄISCHE PATENTANMELDUNG

(21) Anmeldenummer: **82107410.1**

(22) Anmeldetag: **16.08.82**

(51) Int. Cl.³: **C 07 D 501/36**, C 07 D 498/04, A 61 K 31/545 // C07D249/12, (C07D498/04, 265/00, 205/00)

(30) Priorität: **23.09.81 CH 6137/81**

(43) Veröffentlichungstag der Anmeldung: **30.03.83 Patentblatt 83/13**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Montavon, Marc, Dr., Realpstrasse 72, CH-4054 Basel (CH)**
Erfinder: **Reiner, Roland, Dr., Rheinfelderstrasse 8, CH-4058 Basel (CH)**

(74) Vertreter: **Martin, Björn et al, Grenzacherstrasse 124 Postfach 3255, CH-4002 Basel (CH)**

(54) **Cephalosporinderivate, Verfahren zu deren Herstellung und Zwischenprodukte dafür sowie entsprechende pharmazeutische Präparate.**

(57) Leicht hydrolysierbare Ester von Cephalosporinderivaten der allgemeinen Formel

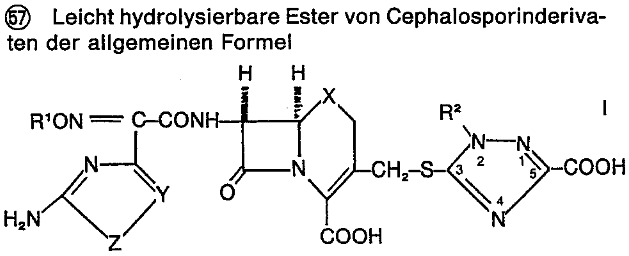

I

in der $R^1$ Wasserstoff, Methyl oder Carboxy-niederes-Alkyl, X Schwefel, Sauerstoff oder eine der Gruppen –SO– und –$SO_2$–, Y die Gruppe –CH= oder Stickstoff, Z Schwefel oder Selen und $R^2$ gegebenenfalls durch Carboxy, Hydroxy, leicht hydrolysierbares Acyloxy oder Dimethylamino substituiertes niederes Alkyl darstellt, mit Ausnahme der (6R, 7R)-7-[2-(2-Amino-4-thiazolyl)-2-methoxyimino-acetamido]-3-[[(5-carboxy-2-methyl-2H-1,2,4-triazol-3-yl)thio]methyl]-8-oxo-5-thia-1-azabicyclo[4.2.0]oct-2-en-2-carbonsäure,

sowie Säureadditionssalze dieser Ester und Hydrate dieser Ester bzw. Säureadditionssalze, Verfahren zu deren Herstellung und Zwischenprodukte dafür sowie wie entsprechende pharmazeutische Präparate.

Die Produkte sind antibiotisch, insbesondere bakterizid, wirksam.

EP 0 075 110 A2

0075110

F.Hoffmann-La Roche & Co. Aktiengesellschaft,Basel,Schweiz

RAN 4410/158

# Cephalosporinderivate, Verfahren zu deren Herstellung und Zwischenprodukte dafür sowie entsprechende pharmazeutische Präparate

Die vorliegende Erfindung betrifft neue Cephalosporinderivate und zwar leicht hydrolysierbare Ester von Verbindungen der allgemeinen Formel

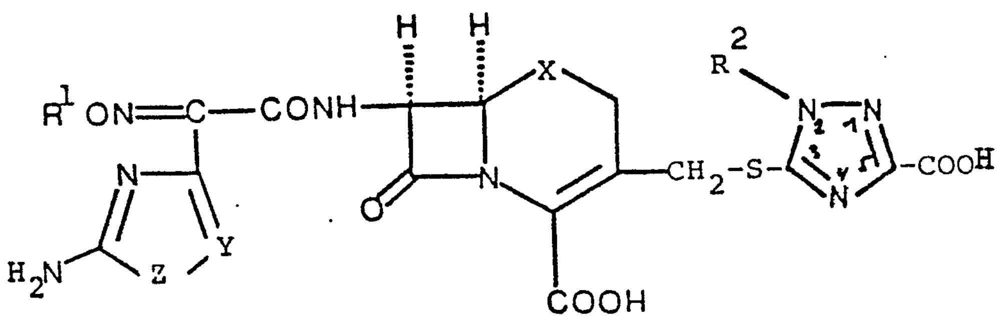

I

in der $R^1$ Wasserstoff, Methyl oder Carboxy-niederes-Alkyl, X Schwefel, Sauerstoff oder eine der Gruppen -SO- und -$SO_2$-, Y die Gruppe -CH= oder Stickstoff, Z Schwefel oder Selen und $R^2$ gegebenenfalls durch Carboxy, Hydroxy, leicht hydrolysierbares Acyloxy oder Dimethylamino substituiertes niederes Alkyl darstellt, mit Ausnahme der (6R,7R)-7-[2-(2-Amino-4-thiazolyl)-2 (methoxyimino)-acetamido]-3-[[(5-carboxy-2-methyl-2H-

Mn/ 22.7.82

1,2,4-triazol-3-yl)thio]methyl]-8-oxo-5-thia-1-aza-bicyclo[4.2.0]oct-2-en-2-carbonsäure,

sowie Säureadditionssalze dieser Ester und Hydrate dieser Ester bzw. Säureadditionssalze.

Der Ausdruck "niederes Alkyl" allein oder in Zusammensetzungen stellt einen geradkettigen oder verzweigten niederen Alkylrest mit bis zu 7, vorzugweise bis zu 4 Kohlenstoffatomen dar; z.B. Methyl, Aethyl, n-Propyl, Isopropyl, n-Butyl, Isobutyl, sec-Butyl, n-Pentyl, n-Heptyl. In den substituierten niederen Alkylresten kann sich der Substituent an beliebiger Stelle des Alkylrestes befinden, wie z.B. in 1-Carboxyäthyl, 2-Carboxyäthyl, 1-Carboxy-1-methyl-äthyl, 2-Carboxy-1-methyl-äthyl, 1-Carboxy-1-methyl-n-propyl, Carboxy-methyl. Ein bevorzugter Carboxy-nieder-alkylrest ist 1-Carboxy-1-methyl-äthyl. Durch leicht hydrolysierbares Acyloxy substituiertes Alkyl kann z.B. Pivaloyloxymethyl, Acetoxymethyl, 1-(Pivaloyloxy)-äthyl, 1-(Aethoxycarbonyloxy)-äthyl und dgl. darstellen.

Als leicht hydrolysierbare Ester der Verbindungen der Formel I sind Verbindungen der Formel I zu verstehen, worin mindestens eine Carboxygruppe in Form einer leicht hydrolysierbaren Estergruppe vorliegt. Beispiele solcher Ester sind die niederen Alkanoyloxyalkylester, z.B. der Acetoxymethyl-, Pivaloyloxymethyl-, 1-Acetoxyäthyl und 1-Pivaloyloxyäthylester; die niederen Alkoxycarbonyloxyalkylester, z.B. der Methoxycarbonyloxymethyl-, 1-Aethoxycarbonyloxyäthyl- und 1-Isopropoxycarbonyloxyäthylester; die Lactonylester, z.B. der Phthalidyl- und Thiophthalidylester; die niederen Alkoxy-nieder-alkylester,z.B. der Methoxymethylester; der gegebenenfalls durch Halogen, niederes Alkyl oder niederes Alkoxy substituierte Phenylester; und die niederen Alkanoylaminomethylester, z.B. der Acetamidomethylester. Auch andere Ester, z.B. die Benzyl- und Cyanmethylester, können brauchbar sein. Die Ester können Mono-, Di-, Tri- oder Tetra-ester sein.

Esterbildung kann in Verbindung mit dem 4-Carboxyrest bzw. dem Carboxyrest der 5-Carboxy-2-$R^2$-2H-1,2,4-triazol-3-yl-gruppe, dem Carboxy-nieder-alkylrest $R^2$ sowie mit der Carboxy-nieder-alkylgruppe $R^1$ auftreten.

Die leicht hydrolysierbaren Ester der Verbindungen der Formel I bilden Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze, wie Sulfate, Nitrate, Phosphate und dgl., Alkyl- und Monoaryl-sulfonate, wie Aethansulfonate, Toluolsulfonate, Benzolsulfonate und dgl. und auch andere organische Säuresalze, wie Acetate, Tartrate, Maleate, Citrate, Benzoate, Salicylate, Ascorbate und dgl.

Die leicht hydrolysierbaren Ester der Verbindungen der Formel I sowie deren Säureadditionssalze können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die erfindungsgemässen Produkte können in der syn-isomeren Form

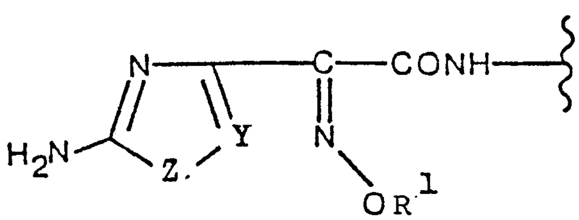

oder in der anti-isomeren Form

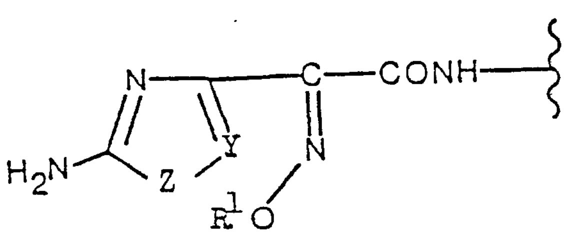

bzw. als Gemische dieser beiden Formen vorliegen. Bevorzugt ist die syn-isomere Form bzw. Gemische, in denen die syn-isomere Form überwiegt.

Interessante Untergruppen der erfindungsgemässen leicht hydrolysierbaren Ester sind diejenigen der folgenden Carbonsäuren

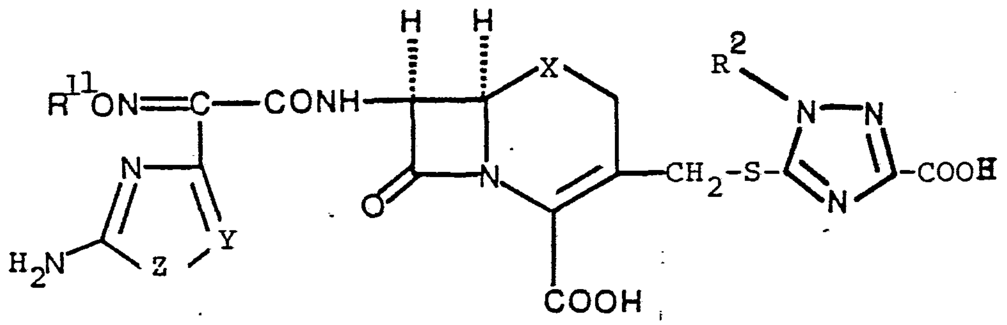

Ia

worin X, Y, Z und $R^2$ die obige Bedeutung haben und $R^{11}$ Wasserstoff oder Carboxy-niederes Alkyl darstellen;

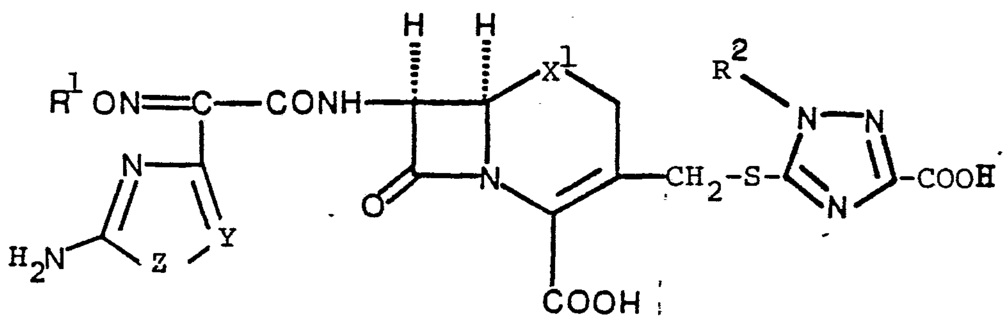

Ib

worin $R^1$, Y, Z, und $R^2$ die obige Bedeutung haben und $X^1$ Sauerstoff oder eine der Gruppen -SO- und $-SO_2-$ darstellt;

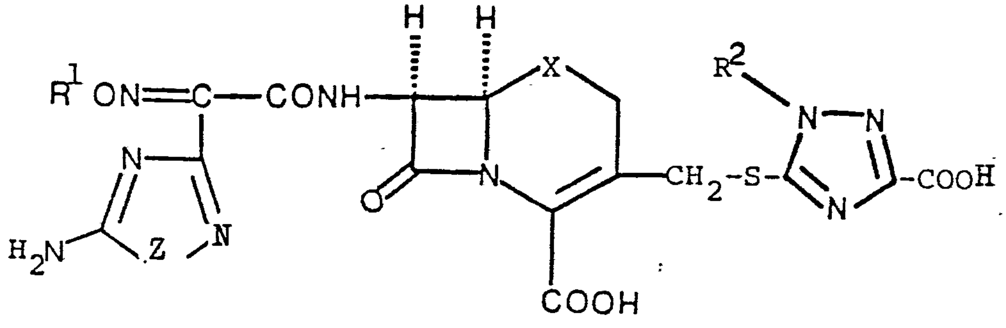

Ic

worin $R^1$, X, Z und $R^2$ die obige Bedeutung haben;

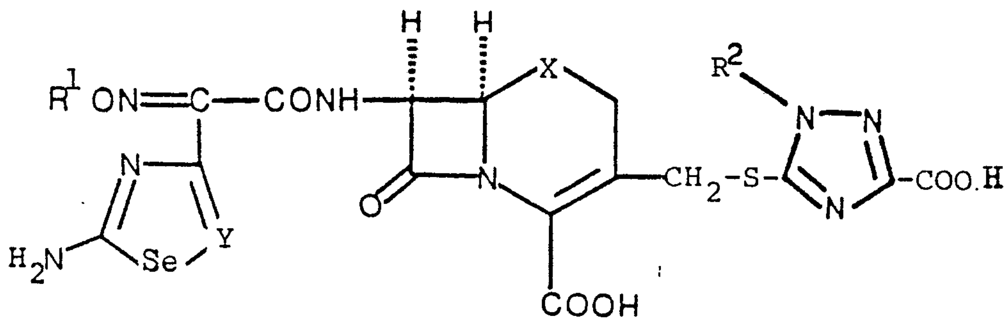

Id

worin $R^1$, X, Y und $R^2$ die obige Bedeutung haben;

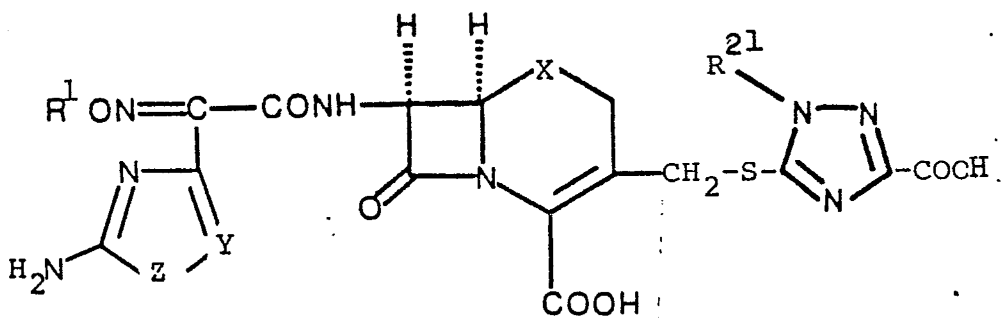

Ie

worin $R^1$, X, Y und Z die obige Bedeutung haben und $R^{21}$ $C_{2-4}$ Alkyl oder durch Carboxy, Hydroxy, leicht hydrolysierbares Acyloxy oder Dimethylamino substituiertes niederes Alkyl darstellt,

und deren Salze sowie die entsprechenden Hydrate.

Die obigen Cephalosporinderivate können erfindungsgemäss dadurch hergestellt werden, dass man die entsprechende Dicarbonsäure, d.h. eine Verbindung der Formel I oder ein Salz dieser Verbindung einer entsprechenden Veresterung unterwirft, erwünschtenfalls ein erhaltenes Produkt S-oxidiert und erwünschtenfalls das erhaltene Produkt in ein Säureadditionssalz oder Hydrat bzw. ein Hydrat dieses Säureadditionssalzes überführt.

Die im obigen Verfahren verwendete Carbonsäure der Formel I kann z.B. dadurch hergestellt werden, dass man

a) zur Herstellung einer Verbindung der Formel I, worin $R^1$ Wasserstoff oder Methyl und Y die Gruppe -CH= darstellt, eine Verbindung der allgemeinen Formel

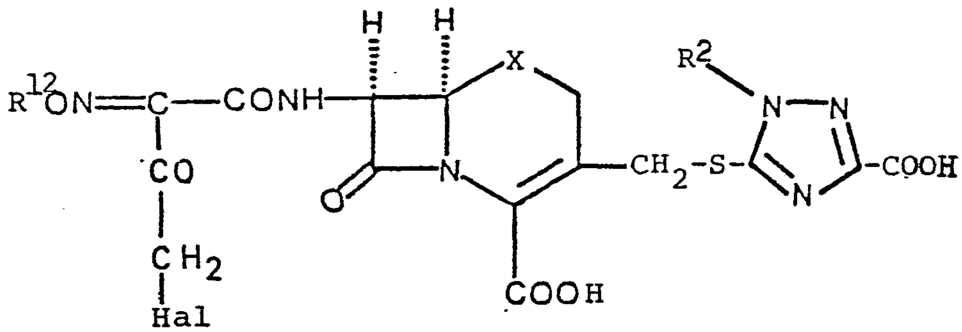

II

in der $R^{12}$ Wasserstoff oder Methyl bedeutet, $R^2$ und X die oben gegebene Bedeutung haben, Hal ein Halogenatom darstellt und die Carboxygruppe in geschützter Form vorliegen kann,

oder ein Salz dieser Verbindung mit Thioharnstoff oder Selenharnstoff umsetzt und gegebenenfalls eine allenfalls vorhandene Carboxyschutzgruppe abspaltet, oder dass man

b) aus einer Verbindung der allgemeinen Formel

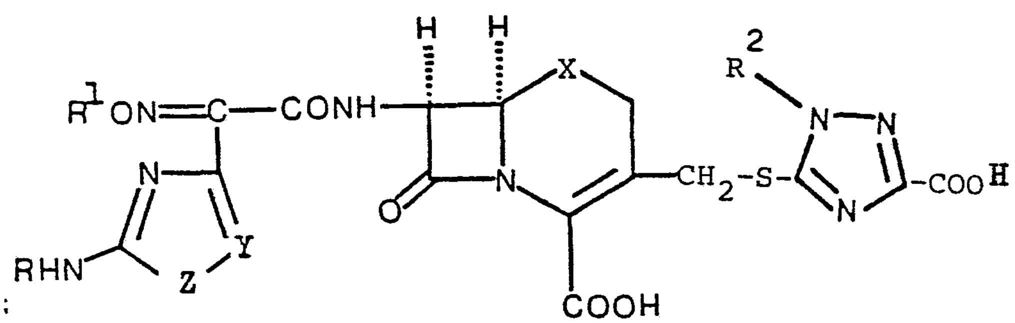

III

in der $R^1$, $R^2$, X, Y und Z die oben gegebene Bedeutung haben, R eine abspaltbare Schutzgruppe darstellt und die Carboxygruppe in geschützter Form vorliegen kann,

oder aus einem Salz dieser Verbindung die Schutzgruppe R und gegebenenfalls eine allenfalls vorhandene Carboxyschutzgruppe abspaltet, oder dass man

c) eine Verbindung der allgemeinen Formel

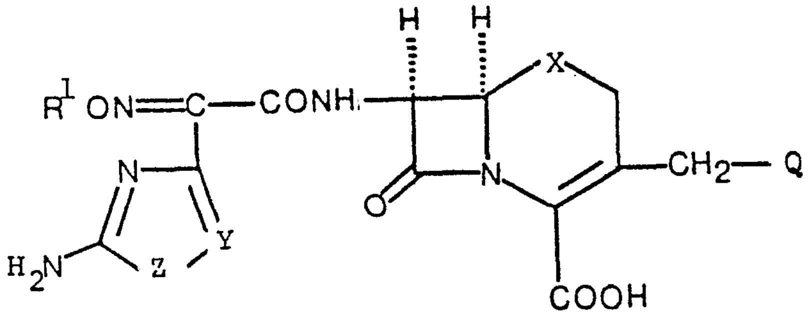

IV

in der $R^1$, X, Y und Z die oben gegebene Bedeutung haben, Q eine austretende Gruppe darstellt und die Carboxygruppe durch Salzbildung mit einer anorganischen oder tertiären organischen Base geschützt sein kann,

oder ein Salz dieser Verbindung in Gegenwart von Wasser mit einem Thiol der Allgemeinen Formel

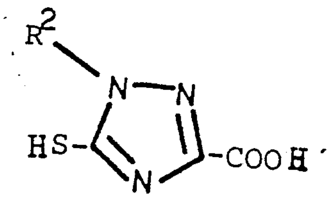

V

in der $R^2$ die oben gegebene Bedeutung hat,

umsetzt, und gegebenenfalls eine allenfalls vorhandene Carboxyschutzgruppe abspaltet, oder dass man

d) ein Verbindung der allgemeinen Formel

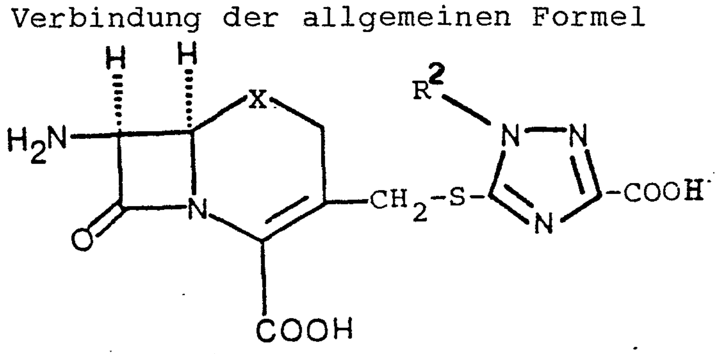

VI

in der X und $R^2$ die oben gegebene Bedeutung haben und die Carboxygruppe und/oder Aminogruppe in geschützter Form vorliegen kann,

mit einem aktivierten Thioester der Formel

0075110

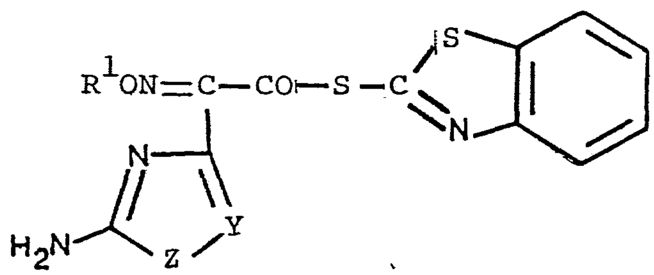

VII

in der $R^1$, Y und Z die oben gegebene Bedeutung haben, umsetzt und gegebenenfalls eine allenfalls vorhandene Carboxyschutzgruppe abspaltet, oder dass man

e) zur Herstellung von Verbindungen der Formel I, worin X eine der Gruppen -SO- und $-SO_2-$ darstellt, eine Verbindung der Formel I, worin X Schwefel oder die Gruppe -SO- darstellt, oder ein Salz dieser Verbindung oxidiert.

Die in den Ausgangsverbindungen der Formeln II und III vorhandene Carboxygruppe in 3-Stellung kann wahlweise geschützt sein, z.B. durch Veresterung zu einem leicht spaltbaren Ester, wie dem Silylester, z.B. dem Trimethylsilylester. Die Carboxygruppe kann auch durch Salzbildung mit einer anorganischen oder tertiären organischen Base, wie Triäthylamin, geschützt werden.

Das erfindungsgemäss eingesetzte Halogenid der Formel II kann, wenn $R^{12}$ Methyl darstellt, z.B. durch Umsetzung einer Verbindung der Formel VI mit einer halogenierten Carbonsäure der allgemeinen Formel

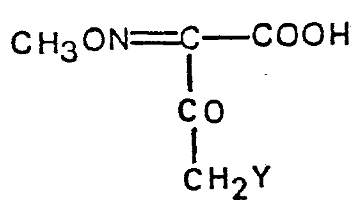

XV

in der Y ein Halogenatom darstellt,

oder mit einem reaktionsfähigen Derivat dieser Verbindung hergestellt werden. Die halogenierte Carbonsäure der Formel XV wird entweder in freier Form eingesetzt in Gegenwart eines Kondensationsmittels, z.B. eines N,N'-disubstituierten Carbodiimides, wie N,N'-Dicyclohexylcarbodiimid, oder einer Azolidverbindung, wie N,N'-Carbonyldiimidazol oder N,N'-Thionyldiimidazol oder auch in Form eines Säurehalogenids, wie des Säurechlorids oder -bromids, in Form eines Säureanhydrids, wie eines Säureanhydrids mit einem Kohlensäuremonoester, z.B. mit Monomethyl- oder Monoisopropylcarbonat, oder in Form eines aktivierten Esters, wie des p-Nitrophenylesters, 2,4-Dinitrophenylesters, N-Hydroxysuccinimidesters oder N-Hydroxyphthalimidesters. Die Reaktion erfolgt im allgemeinen in einem inerten organischen Lösungsmittel, z.B. in einem halogenierten Kohlenwasserstoff, wie Chloroform, Dichlormethan, Tetrachlorkohlenstoff, in einem Aether, z.B. Tetrahydrofuran, Dioxan, in Dimethylformamid, Dimethylacetamid, Wasser oder Mischungen davon. Die Reaktionstemperatur liegt vornehmlich im Bereich von etwa -50 bis +40°C, vorzugsweise bei etwa -10 bis +10°C.

Halogenide der Formel II, worin $R^{12}$ Wasserstoff darstellt, können hergestellt werden, indem man eine Verbindung der Formel VI mit Diketen und dem entsprechenden Halogen (Brom oder Chlor) in das entsprechende 4-Halogenacetoacetamidoderivat der allgemeinen Formel

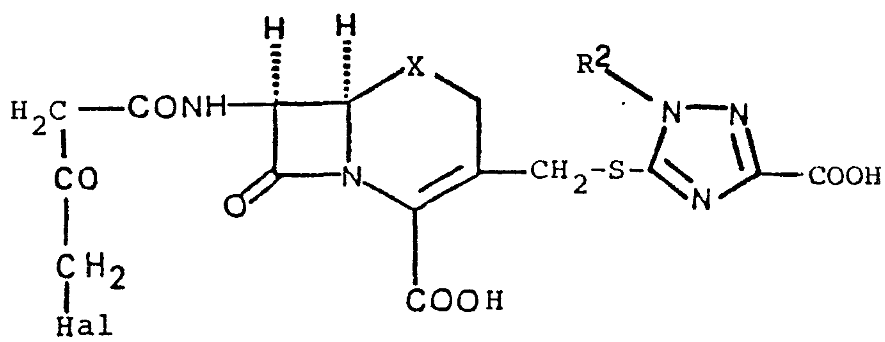

XVI

worin X, $R^2$ and Hal die oben gegebene Bedeutung haben, überführt und dieses Produkt mit einem Nitrosierungsmittel

behandelt.

Die erfindungsgemässe Umsetzung des Halogenids der Formel II bzw. eines Salzes davon mit Thioharnstoff oder Selenharnstoff gemäss obiger Variante a) erfolgt vorzugsweise in einem inerten Lösungsmittel, wie z.B. in einem niederen Alkanol, z.B. Aethanol, in einem niederen Keton, wie Aceton, in einem Aether, wie Tetrahydrofuran oder Dioxan, in Dimethylformamid, Dimethylacetamid, in Wasser oder in Mischungen davon. Die Reaktionstemperatur liegt im allgemeinen im Bereich von etwa 0°C bis 60°C, vorzugsweise wird bei Zimmertemperatur gearbeitet. Als Halogenid der Formel II kann das Chlorid, Bromid, Fluorid oder Jodid eingesetzt werden, bevorzugt verwendet man das Chlorid oder das Bromid. Es kann die freie Säure der Formel II eingesetzt werden, wahlweise aber auch ein Salz davon, wobei die gleichen Salze wie die oben erläuterten Salze der Verbindungen der Formel I in Betracht kommen.

Nach Durchführung der Verfahrensvariante a) kann, falls erwünscht, eine allenfalls im Reaktionsprodukt vorhandene Carboxyschutzgruppe abgespalten werden. Wenn die Schutzgruppe eine Silylgruppe darstellt (Silylester), kann diese Gruppe besonders leicht durch Behandeln des Umsetzungsproduktes mit Wasser abgespalten werden. Wenn die Carboxylgruppe durch Salzbildung (z.B. mit Triäthylamin) geschützt ist, so kann die Abspaltung dieser salzbildenden Schutzgruppe durch Behandlung mit Säure erfolgen. Als Säure kann hierbei z.B. Salzsäure, Schwefelsäure, Phosphorsäure oder Citronensäure verwendet werden.

Die Schutzgruppen R in der für die Variante b) einsetzbaren Ausgangsverbindungen der Formel III sind beispielsweise sauer-hydrolytisch abspaltbare Schutzgruppen, wie z.B. t-Butoxycarbonyl oder Trityl, oder auch basisch-hydrolytisch abspaltbare Schutzgruppen, wie z.B. Trifluoracetyl. Bevorzugte R—Schutzgruppen sind Chlor-, Brom- und Jodacetyl, insbesondere Chloracetyl. Letztere

Schutzgruppen können durch Behandeln mit Thioharnstoff abgespalten werden.

Die Ausgangsverbindungen der Formel III können z.B. durch N-Acylierung der entsprechenden 7-Aminoverbindung hergestellt werden, und zwar indem man eine Verbindung der obigen Formel VI mit einer Säure der allgemeinen Formel

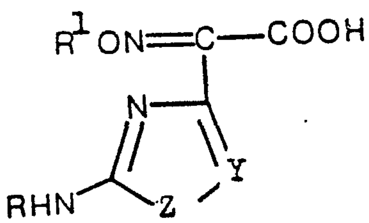

VIII

in der R, $R^1$, Y und Z die oben gegebene Bedeutung haben, oder mit einem reaktionsfähigen funktionellen Derivat dieser Säure umsetzt und eine allenfalls vorhandene Carboxyschutzgruppe gegebenenfalls abspaltet.

Die in der 7-Aminoverbindung der Formel VI vorhandene Carboxygruppe kann wahlweise geschützt sein, und zwar in der oben für die herzustellenden Ausgangsverbindungen der Formeln II und III erläuterten Weise. Die Aminogruppe der Verbindung der Formel III kann z.B. durch eine Silylschutzgruppe, wie Trimethylsilyl, geschützt sein.

Als reaktionsfähige funktionelle Derivate von Säuren der Formel VIII kommen z.B. Halogenide, d.h. Chloride, Bromide und Fluoride; Azide; Anhydride, insbesondere gemischte Anhydride mit stärkeren Säuren; reaktionsfähige Ester, z.B. N-Hydroxysuccinimidester, und Amide, z.B. Imidazolide, in Betracht.

Die Umsetzung der 7-Aminoverbindung der Formel VI mit der Säure der Formel VIII oder einem reaktionsfähigen funktionellen Derivat davon kann in an sich bekannter Weise durchgeführt werden. So kann man z.B. eine freie Säure der Formel VIII mit einem der erwähnten Ester entsprechend

Formel VI mittels eines Carbodiimids, wie Dicyclohexylcarbodiimid, in einem inerten Lösungsmittel, wie Aethylacetat, Acetonitril, Dioxan, Chloroform, Methylenchlorid, Benzol oder Dimethylformamid, kondensieren und anschliessend die Estergruppe abspalten. Anstelle von Carbodiimiden lassen sich als Kondensationsmittel auch Oxazoliumsalze, z.B. N-Aethyl-5-phenyl-isoxazolium-3'-sulfonat, verwenden.

Nach einer anderen Ausführungsform setzt man ein Salz einer Säure der Formel VI,z.B. ein Trialkylammoniumsalz, wie das Triäthylammoniumsalz, mit einem reaktionsfähigen funktionellen Derivat einer Säure der Formel VIII wie oben erwähnt in einem inerten Lösungsmittel, z.B. einem der oben genannten, um.

Nach einer weiteren Ausführungsform wird ein Säurehalogenid, vorzugsweise das Chlorid einer Säure der Formel VIII mit dem Amin der Formel VI umgesetzt. Die Umsetzung erfolgt vorzugsweise in Gegenwart eines säurebindenden Mittels, z.B. in Gegenwart von wässrigem Alkali, vorzugsweise Natronlauge, oder auch in Gegenwart eines Alkalimetallcarbonats, wie Kaliumcarbonat, oder in Gegenwart eines nieder-alkylierten Amins, wie Triäthylamin. Als Lösungsmittel wird vorzugsweise Wasser verwendet, ggf. im Gemisch mit einem inerten organischen Lösungsmittel, wie Tetrahydrofuran oder Dioxan. Es kann auch in einem aprotischen organischen Lösungsmittel, wie z.B. Dimethylformamid, Dimethylsulfoxid oder Hexamethylphosphorsäuretriamid, gearbeitet werden. Bei Verwendung von silylierten Ausgangsverbindungen der Formel VI wird in wasserfreiem Medium gearbeitet.

Die Umsetzung der 7-Aminoverbindung der Formel VI mit der Säure der Formel VIII oder einem reaktionsfähigen funktionellen Derivat davon, kann zweckmässig bei Temperaturen zwischen etwa -40°C und Zimmertemperatur, beispielsweise bei etwa 0-10°C, erfolgen.

Ausgangsverbindungen der Formel III, worin R nicht monohalogeniert ist, wie in Brom-, Chlor- oder Jodacetyl, können auch durch Thiolierung hergestellt werden, und zwar indem man eine Verbindung der allgemeinen Formel

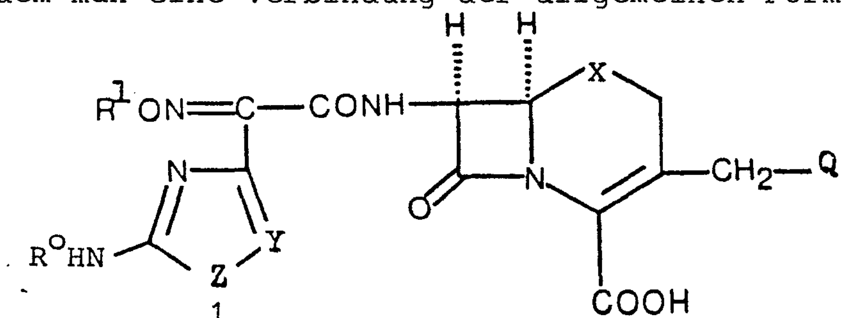

IX

in der $R^1$, X, Y und Z die oben gegebene Bedeutung haben, $R^o$ wie R ist, jedoch nicht monohalogeniert sein kann, Q eine austretende Gruppe darstellt und die Carboxygruppe durch Salzbildung mit einer anorganischen oder tertiären organischen Base geschützt sein kann,

in Gegenwart von Wasser mit einem Thiol der Formel V unsetzt und eine allenfalls vorhandene Carboxyschutzgruppe gegebenenfalls abspaltet.

Als austretende Gruppe Q kommen beispielsweise Halogene, z.B. Chlor, Brom oder Jod, Acyloxyreste, z.B. niedere Alkanoyloxyreste, wie Acetoxy, niedere Alkyl- oder Arylsulfonyloxyreste, wie Mesyloxy oder Tosyloxy, oder der Azidorest in Frage.

Die Umsetzung der Verbindung der Formel IX mit dem Thiol der Formel V kann in an sich bekannter Weise, z.B. bei einer Temperatur zwischen etwa 40 und 80°C, zweckmässig bei etwa 60°C, in Wasser oder in einer Pufferlösung mit einem pH von etwa 6 bis 7, vorzugsweise 6,5, durchgeführt werden. Die Carboxygruppe der erhaltenen Verbindung III kann erwünschtenfalls geschützt werden, z.B. durch Salzbildung oder Veresterung.

7-Aminoverbindungen der Formel VI, können ausgehend von einer Verbindung der Formel

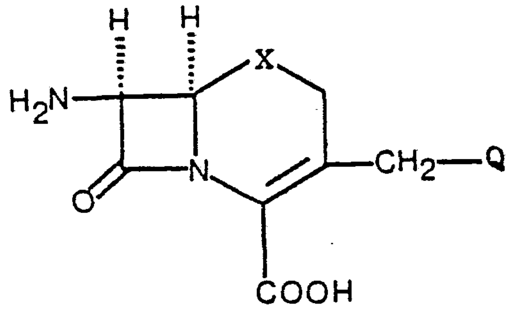

XI

in der X die oben gegebene Bedeutung hat, Q eine austretende Gruppe darstellt und die Carboxygruppe durch Salzbildung mit einer anorganischen oder tertiären organischen Base geschützt sein kann,

in Gegenwart von Wasser mit einem Thiol der Formel V hergestellt werden. Die Umsetzung kann unter den gleichen Bedingungen wie denjenigen erfolgen, wie sie für die Umsetzung der Ausgangsverbindungen IX mit V beschrieben wurden. Andererseits können die Verbindungen der Formel IX ausgehend von einer Verbindung der Formel XI und einer Säure der Formel VIII oder einem reaktionsfähigen funktionellen Derivat davon unter den gleichen Bedingungen hergestellt werden, wie sie für die Umsetzung der Verbindungen der Formeln VI und VIII beschrieben wurden.

Die Carboxygruppe und/oder die Aminogruppe der erhaltenen Verbindung VI können erwünschtenfalls geschützt werden, z.B. durch Veresterung oder Salzbildung der Carboxygruppe oder durch Silylierung.

Säuren der Formel VIII mit Y = -CH= und Z = Se können z.B. durch Umsetzung einer Verbindung der Allgemeinen Formel

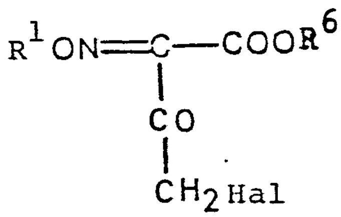

XII

in der $R^1$ und Hal die oben gegebene Bedeutung haben und $R^6$ niederes Alkyl, insbesondere Methyl oder Aethyl, darstellt,

mit Selenharnstoff hergestellt werden, in der gleichen Weise wie die obige Umsetzung der Ausgangsverbindungen der Formel II mit Selenharnstoff. Die so erhaltene Verbindung der Formel

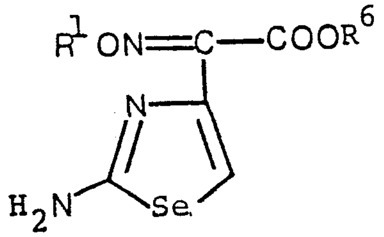
XIII

worin $R^1$ und $R^6$ die oben gegebene Bedeutung haben,

wird anschliessend an der Aminogruppe geschützt, vorzugsweise durch Umsetzung mit dem entsprechenden Halogenid der Formel R-Hal, und die erhaltene Verbindung der Formel

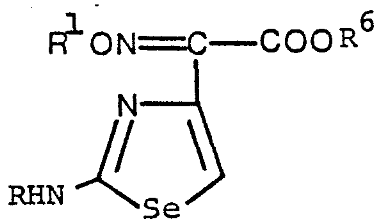
XIV

worin R, $R^1$ und $R^6$ die oben gegebene Bedeutung haben,

zur Abspaltung der Estergruppe $R^6$ sauer oder alkalisch verseift. Die reaktionsfähigen Derivate der so erhaltenen Säuren der Formel VIII werden in an sich bekannter Weise hergestellt.

Die Säuren der Formel VIII mit Y = Stickstoff können gemäss EP 27 599 bzw. (wenn Z = Selen) in Analogie dazu hergestellt werden.

Die Thiole der Formel V stehen in tautomerem Gleichgewicht mit den entsprechenden Thionen und können in an sich bekannter Weise hergestellt werden, beispielsweise aus einem 2-$R^2$-Thio-semicarbazid durch Kochen mit einem

Alkalimetall-nieder-alkanolat, z.B. Natrium-methylat, in einem niederen Alkanol, wie Methanol, und Oxalsäuredimethylester. Wahlweise wird das 2-$R^2$-Thiosemicarbazid in einem inerten Lösungsmittel, wie Acetonitril, mit Oxalsäuremonomethylesterchlorid in Gegenwart eines säurebindenden Mittels erhitzt und das Kondensationsprodukt anschliessend wie oben mit dem Alkalimetall-nieder-alkanolat im niederen Alkanol cyclisiert. Der erhaltene 4,5-Dihydro-1-$R^2$-5-thioxo-1H-1,2,4-triazol-3-carbonsäure-methylester wird ausschliessend in üblicher Weise verseift, z.B. durch Umsetzung mit wässiger Natronlauge und Neutralisation mit Säure.

Gemäss Verfahrensvariante b) wird die Aminoschutzgruppe R einer Ausgangsverbindung der Formel III abgespalten. Durch saure Hydrolyse abspaltbare Schutzgruppen werden vorzugsweise mit Hilfe einer niederen Alkancarbonsäure, die ggf. halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperatur angewendet werden kann, z.B. im Bereiche von etwa 0°C bis +40°C. Alkalisch abspaltbare Schutzgruppen werden im allgemeinen mit verdünnter wässriger Lauge bei 0°C bis 30°C hydrolysiert. Die Chloracetyl-, Bromacetyl- und Jodacetyl-Schutzgruppen können mittels Thioharnstoff in saurem, neutralem oder alkalischem Milieu bei etwa 0-30°C abgespalten werden. Hydrogenolytische Abspaltung (z.B. Abspaltung von Benzyl) ist ungeeignet, da bei der Hydrogenolyse die Oximfunktion zur Aminogruppe reduziert wird.

Die allenfalls vorhandene Carboxyschutzgruppe kann in der gleichen Weise wie oben für die Verfahrensvariante a) beschrieben abgespalten werden. Die Carboxyschutzgruppe kann in der gleichen Weise auch vor der Abspaltung der Schutzgruppe R abgespalten werden.

Die für die Verfahrensvariante c) einsetzbaren Ausgangsverbindungen der allgemeinen Formel IV können durch Abspaltung der Aminoschutzgruppe $R^{O}$ der oben beschriebenen Verbindungen der Formel IX erhalten werden. Diese Abspaltung kann in der gleichen Weise durchgeführt werden wie die oben beschriebene Abspaltung der Aminoschutzgruppen R der Ausgangsverbindungen der Formel III.

Als austretende Gruppe Q einer Verbindung der Formel IV kommen beispielsweise Halogene, z.B. Chlor, Brom oder Jod, Acyloxyreste, z.B. niedere Alkanoyloxyreste, wie Acetoxy, niedere Alkyl- oder Arylsulfonyloxyreste, wie Mesyloxy oder Tosyloxy, oder der Azidorest in Frage.

Die Umsetzung der Verbindung der Formel IV mit dem Thiol der Formel V kann in an sich bekannter Weise, z.B. bei einer Temperatur zwischen etwa 40 und 80°C, zweckmässig bei etwa 60°C, in Wasser oder in einer Pufferlösung mit einem pH von etwa 6 bis 7, vorzugsweise 6,5, durchgeführt werden. Die allenfalls vorhandene Carboxyschutzgruppe kann in der gleichen Weise wie oben für die Verfahrensvariante a) beschrieben abgespalten werden.

Erwünschtenfalls kann bei der Umsetzung der Verbindungen IV und V bzw. IX und V bzw. XI und V anstelle einer Verbindung V ein leicht hydrolysierbarer Ester davon eingesetzt werden, wobei Zwischenprodukte der allgemeinen Formel

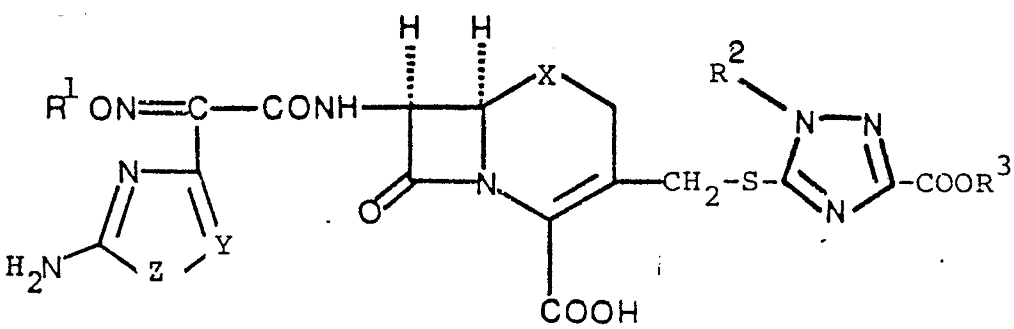

X

worin $R^1$, X, Y, Z und $R^2$ die oben gegebene Bedeutung haben und $COOR^3$ eine leicht hydrolysierbare Estergruppe darstellt,

erhalten werden.

Diese Verbindungen sind als wirksame Metaboliten nach der oralen Verabreichung der erfindungsgemässen Ester anzusehen.

Der für die Variante d) verwendete aktivierte Ester VII kann aus der Carbonsäure VIII (ggf. geschützt wie oben erläutert) durch Umsetzung mit 2,2'-Dithiobisbenzothiazol in Gegenwart von Triphenylphosphin oder eines Tri-niederalkylphosphits bei Raumtemperatur in einem inerten organischen Lösungsmittel, wie z.B. Acetonitril oder Methylenchlorid, hergestellt werden. Die anschliessene Umsetzung der Verbindungen VI und VII wird vorzugsweise in einem inerten Lösungsmittel oder in Gemisch mit Wasser, z.B. in Methylenchlorid oder Aethylacetat, bei etwa Raumtemperatur, vorzugsweise in Gegenwart einer Base, wie Triäthylamin, ausgeführt.

Eine allenfalls noch vorhandene Carboxyschutzgruppe kann anschliessend, wie oben für die Verfahrentvariante a) bereits erläutert, abgespalten werden.

Die Variante e), die Oxidation von Verbindungen der Formel I, worin X Schwefel oder die Gruppe -SO- darstellt, bzw. eines Salzes davon, erfolgt durch Behandlung mit einem organischen oder anorganischen Oxidationsmittel. Als Oxidationsmittel dienen verschiedene, Sauerstoff leicht abgebende Verbindungen, wie z.B. organische Peroxide, z.B. monosubstituierte organische Peroxide, wie $C_1$-$C_4$ Alkyl- oder Alkanoylhydroperoxide, wie t-Butylhydroperoxid, Perameisensäure, Peressigsäure; sowie phenylsubstituierte Derivate dieser Hydroperoxide, wie Cumolhydroperoxid, Perbenzoesäure. Der Phenylsubstituent kann gegebenenfalls eine weitere niedere Gruppe, z.B. $C_1$-$C_4$ Alkyl- oder Alkoxy-, Halogen- oder Carboxygruppe tragen, z.B. 4-Methylperbenzoesäure, 4-Methoxyperbenzoesäure, 3-Chlorperbenzoesäure, Monoperphthalsäure. Als Oxidationsmittel können auch

verschiedene anorganische Oxidationsmittel verwendet werden, z.B. Wasserstoffperoxid; Ozon; Permanganate, wie Kalium- oder Natriumpermanganat; Hypochlorite, wie Natrium-, Kalium- oder Ammoniumhypochlorit; Peroxymono- und Peroxydischwefelsäure. Bevorzugt ist die Verwendung von 3-Chlorperbenzoesäure. Die Oxidation erfolgt mit Vorteil in einem inerten Lösungsmittel, z.B. in einem aprotischen inerten Lösungsmittel, wie Tetrahydrofuran, Dioxan, Methylenchlorid, Chloroform, Aethylacetat oder Aceton; oder in einem protischen Lösungsmittel, wie Wasser, einem niederen Alkanol, z.B. Methanol, Aethanol, oder einer niederen, ggf. halogenierten Alkancarbonsäure, z.B. Ameisensäure, Essigsäure, Trifluoressigsäure. Die Reaktionstemperatur bewegt sich vornehmlich im Bereiche von -20°C bis +50°C.

Bei Verwendung von äquimolaren Mengen Oxidationsmittel bzw. leichtem Ueberschuss im Verhältnis zur Ausgangsverbindung der Formel I, worin X Schwefel darstellt, bzw. einem Salz davon, erhält man vornehmlich das entsprechende Sulfoxid der Formel I, worin X die Gruppe -SO- darstellt. Erhöht man die Menge des Oxidationsmittels auf das doppelte stöchiometrische Verhältnis oder mehr, bildet sich das entsprechende Sulfon der Formel I, worin X die Gruppe $-SO_2-$ darstellt. Es ist ebenfalls möglich, das Sulfon der Formel I aus dem entsprechenden Sulfoxid durch Behandlung mit dem Oxidationsmittel in äquimolarer oder grösserer Menge zu erhalten. Die Verfahrensbedingungen sind im wesentlichen die gleichen wie bei der Herstellung des Sulfoxids.

Die erhaltene Carbonsäure der Formel I kann anschliessend in ein Salz übergeführt werden, z.B. in ein Säureadditionssalz wie oben beschrieben, oder auch in Salze mit Basen, z.B. Alkalimetallsalze, wie das Natrium- und Kaliumsalz; das Ammoniumsalz; Erdalkalimetallsalze, wie das Calcium-salz; Salze mit organischen Basen, wie Salze mit Aminen, z.B. Salze mit N-Aethyl-piperidin, Procain, Dibenzylamin, N,N'-Dibenzyläthylendiamin, Alkylaminen

oder Dialkylaminen, sowie Salze mit Aminosäuren, wie z.B. Salze mit Arginin oder Lysin. Die Salze können Mono-, Di-, Tri- oder Tetrasalze sein. Die Salze werden in an sich bekannter Weise hergestellt, z.B. durch Umsetzen der Carbonsäure der Formel I mit einer äquivalenten Menge der gewünschten Säure bzw. Base, zweckmässig in einem Lösungsmittel, wie Wasser oder in einem organischen Lösungsmittel, wie Aethanol, Methanol, Aceton und anderen mehr. Die Temperatur der Salzbildung ist nicht kritisch. Sie liegt im allgemeinen bei Raumtemperatur, kann aber auch leicht darüber oder darunter, etwa in Bereiche von 0°C bis +50°C, sein.

Zur erfindungsgemässen Herstellung der leicht hydrolysierbaren (Mono-, Di-, Tri- oder Tetra-) Ester der Carbonsäuren der Formel I wird die Carbonsäure bzw. eines ihrer Salze vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat oder einem organischen Amin, wie Triäthylamin, beschleunigt werden. Je nach der verwendeten Menge Veresterungsmittel erhält man Mono-, Di-, Tri- oder Tetraester. Für die vollständige Veresterung einer Säure der Formel I verwendet man vorzugsweise einen Ueberschuss an dem entsprechenden Halogenid. Die Veresterungsreaktion wird vorzugsweise in einem inerten organischen Lösungsmittel, ggfs. in Gemisch mit Wasser, durchgeführt, wie in Dimethylacetamid, Hexamethylphosphorsäuretriamid, Dimethylsulfoxid oder, vorzugsweise, in Dimethylformamid. Die Temperatur liegt vorzugsweise im Bereiche von etwa 0-40°C.

Ein erhaltener Ester kann erwünschtenfalls S-oxidiert werden, wobei die Gruppe X = Schwefel gegen -SO- bzw. $-SO_2-$ ausgetauscht wird. Die Oxidation kann in der oben beschriebenen Weise erfolgen.

Die Herstellung der Säureadditionssalze und Hydrate

der Ester der Verbindungen der Formel I bzw. der Hydrate dieser Säureadditionssalze kann in an sich bekannter Weise erfolgen, die Säureadditionssalze z.B. durch Umsetzung eines Esters einer Carbonsäure der Formel I mit einer äquivalenten Menge der gewünschten Säure, zweckmässig in einem Lösungsmittel, wie Wasser oder in einem organischen Lösungsmittel, wie Aethanol, Methanol, Aceton und anderem mehr. Die Temperatur der Salzbildung ist nicht kritisch. Sie liegt im allgemeinen bei Raumtemperatur, kann aber auch leicht darüber oder darunter, etwa im Bereiche von 0°C bis +50°C, sein.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt einer feuchten Atmosphäre, z.b. bei etwa +10°C bis +40°C, ausgesetzt werden.

Ein allenfalls erhaltenes syn/anti-Gemisch kann in die entsprechenden syn- und anti-Formen in üblicher Weise aufgetrennt werden, beispielsweise durch Umkristallisation oder durch chromatographische Methoden unter Verwendung eines geeigneten Lösungsmittels bzw. Lösungsmittelgemisches. Vorzugsweise erhält man aber die erfindungsgemässen Ester in der syn-isomeren Form, indem man eine Ausgangsverbindung der Formel IV in syn-Form einsetzt.

Die erfindungsgemässen Ester der Verbindungen der Formel I sowie die Produkte der Formeln I und X und die entsprechenden Salze bzw. die Hydrate dieser Produkte sind antibiotisch, insbesondere bakterizid wirksam. Sie besitzen ein breites Wirkungsspektrum gegen Grampositive Bakterien, z.B. Streptokokken, und gegen Gram-negative Bakterien, einschliesslich β-Lactamase-bildende Gram-negative Erreger, wie Escherichia coli, Serratia

marcescens und Klebsiella pneumoniae.

Die erfindungsgemässen Ester der Verbindungen der Formel I sowie die Produkte der Formeln I und X und die entsprechenden Salze bzw. die Hydrate dieser Produkte können zur Behandlung und Prophylaxe von Infektionskrankheiten verwendet werden. Für den Erwachsenen kommt eine Tagesdosis von etwa 0,1 g bis etwa 4 g, insbesondere etwa 0,25 g bis etwa 2 g in Betracht. Enterale oder parenterale Verabreichung ist möglich; die leicht hydrolysierbaren Ester der Verbindungen der Formel I sowie die entsprechenden Säureadditionssalze und Hydrate besitzen den besonderen Vorzug der Verwendbarkeit für die enterale, z.B. die orale, Verabreichung.

Die orale antimikrobielle Wirksamkeit der erwähnten Produkte kann anhand von in-vivo-Versuchen an der Maus nachgewiesen werden. Im nachstehenden bedeutet die kurative Dosis ($CD_{50}$, p.o., mg/kg) diejenige perorale Dosis, bei der 50% der getesteten Mäuse überleben.

Testverbindungen:

Produkt A: Methylen-(5S,6R,7R)-7-[2-(2-Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]thio]methyl]-8-oxo-5-thia-1-azabicyclo[4.2.0]oct-2-en-2-carboxylat-pivalat-5-oxid

- Cephalexin (vorbekanntes, anerkannt oral wirksames Cephalosporin)

Ergebnis ($CD_{50}$, p.o., mg/kg)

| Erreger | Produkt A | Cephalexin |
|---|---|---|
| Escherichia coli | 1.0 | 3,2 |
| Serratia marcescens | 7,5 | > 50 |
| Klebsiella pneumoniae | 8,8 | |
| Streptocccoccus pyogenes | > 12 | 1,8 |

Die erfindungsgemässen Produkte können als Heilmittel, z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaesthetica oder Puffer. Sie können auch noch andere therapeutisch wertvolle Stoffe enthalten.

## Beispiel 1

Herstellung von Methylen (5S,6R,7R)-7-[2-(2-Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]thio]methyl]-8-oxo-5-thia-1-azabicyclo-[4.2.0]oct-2-en-2-carboxylat-pivalat-5-oxid.

2,35 g Methylen- (6R,7R)-7-[2-(2-Amino-4-thiazolyl)-2-[(Z)-methoxyimino]-acetamido]-3-[[[2-methyl-5[[(pivaloyl-oxy)methoxy]carbonyl]-2H-1,2,4-triazol-3-yl]-thio]methyl]-8-oxo-5-thia-1-azabicyclo[4.2.0]oct-2-en-2-carboxylat-pivalat werden in 30 ml Chloroform gelöst und bei 20°C portionenweise mit 0,61 g m-Chlorperbenzoesäure (85%ig) während ca. 30 Min. versetzt. Das Gemisch wird mit 150 ml Aethylacetat verdünnt und 2mal mit 50 ml 5%iger wässriger Natriumhydrogencarbonat-Lösung gewaschen, mit Natriumsulfat getrocknet und eingedampft. Man erhält einen Eindampfrückstand als dunkles Harz. Zwecks Reinigung wird dieser an einer Kieselgelsäule mit Aethylacetat als Elutionsmittel chromatographiert. Man erhält nach dem Fällen aus Aethylacetat/tiefs. Petroläther reine Titelsubstanz mit $[\alpha]_D^{25}$ = -98° (c = 1 in Aceton). Die Mikroanalyse und das Kernresonanzspektrum entsprechen der angegebenen Struktur.

Das als Ausgangsmaterial verwendete Methylen-(6R,7R)-7-[2-(2-Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]thio]methyl]-8-oxo-5-thia-1-azabicyclo-[4.2.0]oct-2-en-2-carboxylat-pivalat kann wie folgt hergestellt werden :

a) Herstellung von 4,5-Dihydro-1-methyl-5-thioxo-1H-1,2,4-triazol-3-carbonsäure-methylester:

12,9 g 1-Methoxyoxalyl-2-methyl-thiosemicarbazid werden einer Lösung von 1,6 g Natrium in 200 ml Methanol zugegeben. Das Gemisch wird 4 Stunden unter Rückfluss gekocht. Die nach einer 1/2 Stunde ausgefallene Substanz wird abgetrennt. Die Mutterlauge wird im Vakuum bei 40°C eingedampft. Der Eindampfrückstand wird in 100 ml Wasser gelöst und mit konz. Salzsäure angesäuert. Das dabei ausgeschiedene Kristallisat liefert nach dem Umkristallisieren aus Wasser reinen, farblosen 4,5-Dihydro-1-methyl-5-thioxo-1H-1,2,4-triazol-3-carbonsäure-methylester vom Schmelzpunkt 188-190°C (Zers.).

b) Herstellung von 4,5-Dihydro-1-methyl-5-thioxo-1H-1,2,4-triazol-3-carbonsäure:

3,46 g 4,5-Dihydro-1-methyl-5-thioxo-1H-1,2,4-triazol-3-carbonsäure-methylester werden in 50 ml 1N Natronlauge gelöst. Nach halbstündigem Rühren bei 25°C wird die Reaktionslösung mit 25 ml 2N Salzsäure sauer gestellt und im Vakuum bei 40°C eingeengt, wobei die gewünschte Säure kristallisiert. Diese wird abgenutscht, mit Eiswasser gewaschen und im Hochvakuum bei 45°C getrocknet. Man erhält farblose 4,5-Dihydro-1-methyl-5-thioxo-1H-1,2,4-triazol-3-carbonsäure vom Schmelzpunkt 177-178°C (Zers.).

c) Herstellung von (6R,7R)-7-amino-3-[[(5-carboxy-2-methyl-2H-1,2,4-triazol-3-yl)thio]methyl]-8-oxo-5-thia-1-azabicyclo[4.2.0]oct-2-en-2-carbonsäure:

34 g 7-Aminocephalosporansäure werden zusammen mit 25,5 g 4,5-Dihydro-1-methyl-5-thioxo-1H-1,2,4-triazol-3-carbonsäure in 500 ml Wasser suspendiert. Das pH wird mit 3N Natronlauge auf 6,5 gestellt. Das Gemisch wird 4 Stunden bei 55°C unter Stickstoffbegasung gerührt, wobei das pH mit 3N Natronlauge mit Hilfe eines Autotitrators konstant bei 6,5 gehalten wird. Die dunkle Lösung wird auf 25°C gekühlt und mit konz. Salzsäure auf pH 3,5 gestellt. Nach 15 Minuten wird das ausgefallene Material abgenutscht, in 500 ml Wasser suspendiert und mit 3N Natronlauge bei pH 7 in Lösung gebracht. Die orangerote Lösung wird mit konz. Salzsäure auf pH 3 gestellt. Die ausgefallene Substanz

0075110

wird abgenutscht und nacheinander mit 250 ml Wasser, 500 ml Aceton, 500 ml Tiefsiedendem Petroläther gewaschen und über Nacht im Vakuum bei 40-45°C getrocknet. Man erhält (6R,7R)-7-Amino-3-[[(5-carboxy-2-methyl-2H-1,2,4-triazol-3-yl)thio]methyl]-8-oxo-5-thia-1-azabicyclo[4.2.0]oct-2-en-2-carbonsäure als beige gefärbtes Pulver.

d) Herstellung von (6R,7R)-3-[[(5-carboxy-2-methyl-2H-1,2,4-triazol-3-yl)thio]methyl]-7-[2-(2-chloracetamido)-4-thiazolyl]-2-[(Z)-methoxyimino]acetamido]-8-oxo-5-thia-1-azabicyclo[4.2.0]oct-2-en-2-carbonsäure:

Zu 600 ml über Calciumchlorid getrocknetem Methylenchlorid werden 75 g Phosphorpentachlorid gegeben. Die Suspension wird unter Rühren auf -10°C abgekühlt, und während ca. 5 Minuten werden 138 ml N,N-Dimethylacetamid zugetropft, wobei die Temperatur auf -5°C ansteigt. Die Suspension wird auf -15°C gekühlt und 15 Minuten gerührt. Nach dem Abkühlen auf -20°C werden 100 g 2-(2-chloracetamido-4-thiazolyl)-2-(Z)-methoxyimino-essigsäure zugegeben und 45 Minuten bei -15°C gerührt, wibei eine gelb-orange Lösung entsteht. Diese Lösung wird auf -30°C gekühlt, mit 150 g Eis versetzt und 10 Minuten bei -5°C gerührt. Die das gebildete Säurechlorid enthaltende Methylenchlorid-Phase wird abgetrennt und zunächst bei ca. -15°C aufbewahrt. Diese Säurechlorid-Lösung wird einer auf 0-5°C gedühlten Lösung, die durch Versetzen von 111,5 g (6R,7R)-7-Amino-3-[[(5-carboxy-2-methyl-2H-1,2,4-triazol-3-yl)thio]methyl]-8-oxo-5-thia-1-azabicyclo[4.2.0]oct-2-en-2-carbonsäure mit 3N Natronlauge bis pH 8,2 in 500 ml Wasser hergestellt wurde, während 30 Minuten unter kräftigem Rühren zugetropft, wobei der pH-Wert mit 3N Natronlauge mit Hilfe eines Autotitrators bei 8,0-8,2 gehalten wird. Das Gemisch wird 2 Stunden bei 25°C gerührt. Danach werden 900 ml Methylenchlorid und 6 l n-Butanol zugegeben. Unter kräftigem Rühren wird das pH mit 3N Salzsäure auf 2 zurückgestellt. Die organische Phase wird abgetrennt, dreimal mit je 3 l Wasser gewaschen, mit 60 g Entfärbungskohle 15 Minuten

lang verrührt und filtriert. Das hellgelbe Filtrat wird im Vakuum bei 60°C stark eingeengt, wobei das Reaktionsprodukt ausfällt. Letzteres wird abgenutscht und nacheinander mit n-Butanol, Aether und tiefsiedendem Petroläther gewaschen und über Nacht im Vakuum bei 40°C getrocknet. Man erhält rohe, beige (6R,7R)-3-[[(5-Carboxy-2-methyl-2H-1,2,4-triazol-3-yl)thio]methyl]-7-[2-(2-chloracetamido)-4-thiazolyl]-2-[(Z)-methoxyimino]-acetamido]-8-oxo-5-thia-1-azabicyclo[4.2.0]oct-2-en-2-carbonsäure.

e) Herstellung des Dinatriumsalzes der (6R,7R)-7-[2-(2-Amino-4-thiazolyl)-2-[(Z)-methoxyimino]acetamido]-3-[[(5-carboxy-2-methyl-2H-1,2,4-triazol-3-yl)thio]-methyl]-8-oxo-5-thia-1-azabicyclo[4.2.0]oct-2-en-2-carbonsäure:

53 g (6R,7R)-3-[[(5-carboxy-2-methyl-2H-1,2,4-triazol-3-yl)thio]methyl]-7-[2-(2-chloracetamido)-4-thiazolyl]-2-[(Z)-methoxyimino]acetamido]-8-oxo-5-thia-1-azabicyclo-[4.2.0]oct-2-en-2-carbonsäure werden in 1 l Wasser zusammen mit 34 g Thioharnstoff suspendiert. Das pH wird durch Zutropfen von 1N Natronlauge auf 7 gestellt, wobei eine dunkelbraune Lösung entsteht. Diese wird über Nacht unter Stickstoffbegasung bei pH 7 gerührt, wobei das pH mit 1N Natronlauge mit Hilfe eines Autotitrators konstant gehalten wird. Das pH der Reaktionslösung wird mit 1N Salzsäure auf 3,5 unter Rühren zurückgestellt. Die ausgefallene Substanz (Fraktion I) wird abgenutscht, mit 250 ml Wasser gewaschen und im Vakuum bei 40°C getrocknet. Die Fraktion I ist braun gefärbt und stark verunreinigt; sie wird verworfen. Die orange Mutterlauge wird mit 1N Salzsäure unter Rühren auf pH 2,65 zurückgestellt. Das ausgefallene Material wird abgenutscht und mit 250 ml Wasser gewaschen. Das beige-farbene Nutschgut wird unter vermindertem Druck mit Aethanol azeotropiert, abgenutscht, mit Aethanol und tiefsiedendem Petroläther gewaschen und im Vakuum bei 40°C getrocknet. Man erhält so eine beige-braune Fraktion II, welche zwecks Reinigung und Ueberführung in das Dinatriumsalz in 2 l Methanol suspendiert, mit 50 ml einer 2N Lösung des Natriumsalzes der 2-Aethylcapronsäure in Aethylacetat versetzt und anschliessend mit 200 ml wasser 30 Minuten gerührt wird. Wenig ungelöstes, braunes Material wird abfiltriert und verworfen. Vom orange gefärbten Filtrat wird im Vakuum bei 40°C ca. 1 l abgedampft, dann 1 l Aethanol zugegeben und im Vakuum auf ein Volumen von ca. 500 ml eingeengt. Vom dabei ausgefallenen, schmierigen braunen Harz, welches ein starkes Gemisch darstellt, wird abdekantiert. Die abdekantierte gelborange Lösung wird mit 1 l Aethanol versetzt und im Vakuum bei 40°C stark eingeengt, wobei teilweise Substanz ausfällt. Nach Zugabe von 2,5 l Methanol entsteht eine gelbe Lösung, welche im Vakuum bei 40°C stark eingeengt wird. Das dabei ausgefallene Dinatriumsalz wird abgenutscht, mit Methanol und tiefsiedendem Petroläther gewaschen und im Hochvakuum bei 35°C getrocknet. Man erhält reines, beige-farbenes Dinatriumsalz der (6R,7R)-7-[2-(2-Amino-4-thiazolyl)-2-[(Z)-methoxyimino]acetamido]-3-[[(5-carboxy-2-methyl-2H-1,2,4-triazol-3-yl)thio]methyl]-8-oxo-5-thia-1-azabicyclo[4.2.0]oct-2-en-2-carbonsäure mit $[\alpha]_D^{25} = -38,7°$ (c = 1 in Wasser).

f) Herstellung von Methylen-(6R,7R)-7-[2-(2-Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]-thio]methyl]-8-oxo-5-thia-1-azabicyclo[4.2.0]oct-2-en-2-carboxylat-pivalat:

3,9 g des Dinatriumsalzes des (6R,7R)-7-[2-(2-Amino-4-thiazolyl)-2-[(Z)-methoxyimino]acetamido]-3-[[5-carboxy-2-methyl-2H-1,2,4-triazol-3-yl)thio]methyl]-8-oxo-5-thia-1-azabicyclo[4.2.0]oct-2-en-2-carbonsäure werden in einem Gemisch von 100 ml Dimethylformamid und 25 ml Wasser gelöst. Diese Lösung wird bei 0-5°C unter Stickstoff-Begasung mit 3,13 g Jodmethylpivalat versetzt. Das Reaktionsgemisch wird 30 Minuten bei 0-5°C gerührt, danach auf 500 ml Wasser gegossen und dreimal mit je 200 ml Aethylacetat extrahiert. Die wässrige Phase wird verworfen. Die vereinigten Aethylacetatphasen werden nacheinander mit Wasser, verdünnter wässriger Natriumhydrogencarbonatlösung und nochmals mit Wasser gewaschen, über Natriumsulfat getrocknet und im Vakuum bei 40°C stark eingeengt. Nach Zugabe von tiefsiedendem Petroläther wird das ausgefallene Rohprodukt abgenutscht, mit tiefsiedendem Petroläther gewaschen und im Hochvakuum bei 40°C getrocknet. Man erhält beigefarbenes, amorphes Rohprodukt. Zwecks Reinigung wird dieses Produkt an einer Säule mit 100 g Kieselgel (0,2-0,5 mm) und Aethylacetat als Elutionsmittel chromatographiert. Man erhält reine, beigefarbene, amorphe Titelsubstanz mit $R_f$ = 0,31 in der Dünnschichtchromatographie auf Kieselgel-60F-254-Fertigplatten mit Aethylacetat als Laufmittel. Die spezifische Drehung beträgt $[\alpha]_D^{25}$ = -121,6° (c = 0,4924 in Aceton); das Kernresonanzspektrum und die Mikroanalyse entsprechen der angegebenen Struktur.

## Beispiel 2

Es wird in üblicher Weise eine Gelatine-Steckkapsel folgender Zusammensetzung hergestellt:

| | |
|---|---|
| Methylen-(5S,6R,7R)-7-[2-(2-Amino-4-thiazolyl)-2-[(Z)-methoxyimino]acetamido]-3-[[[2-methyl-5-[[(pivaloyloxy)methoxy]carbonyl]-2H-1,2,4-triazol-3-yl]thio]methyl]-8-oxo-5-thia-1-azabicyclo[4.2.0]oct-2-en-2-carboxylat-pivalat-5-oxid | 500 mg |
| Luviskol (wasserlösliches Polyvinylpyrrolidon) | 20 mg |
| Mannit | 20 mg |
| Talk | 15 mg |
| Magnesiumstearat | 2 mg |
| | 557 mg |

Beispiel 3

Es wird in üblicher Weise eine Tablette folgender Zusammensetzung hergestellt:

| | |
|---|---|
| Methylen-(5S,6R,7R)-7-[2-(2-Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]thio]methyl]-8-oxo-5-thia-1-azabicyclo[4.2.0]oct-2-en-2-carboxylat-pivalat-5-oxid | 250 mg |
| Lactose | 70 mg |
| Maisstärke | 65 mg |
| Polyvinylpyrrolidon | 10 mg |
| Magnesiumstearat | 5 mg |
| | 400 mg |

Mit dem Wirkstoff, der Lactose, dem Polyvinylpyrrolidon und 40 Gewichtsteilen der Maisstärke wird in üblicher Weise ein Granulat hergestellt. Dieses wird mit den restlichen 25 Gewichtsteilen Maisstärke und 5 Gewichtsteilen Magnesiumstearat vermischt und zu Tabletten gepresst.

Patentansprüche

1. Leicht hydrolysierbare Ester von Cephalosporinderivaten der allgemeinen Formel

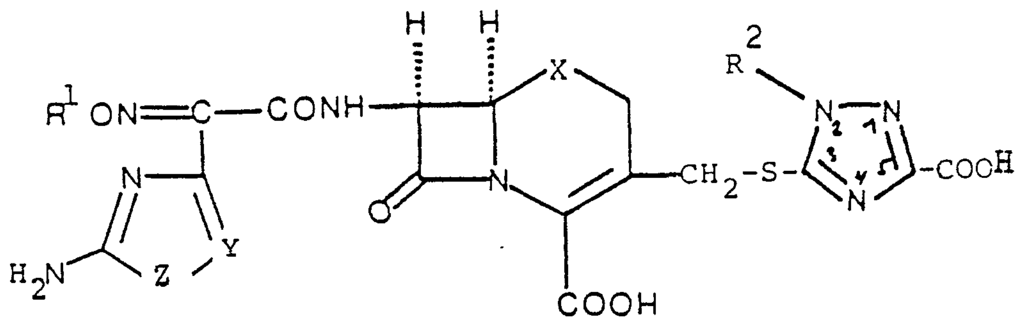

I

in der $R^1$ Wasserstoff, Methyl oder Carboxy-niederes-Alkyl, X Schwefel, Sauerstoff oder eine der Gruppen -SO- und $-SO_2-$, Y die Gruppe -CH= oder Stickstoff, Z Schwefel oder Selen und $R^2$ gegebenenfalls durch Carboxy, Hydroxy, leicht hydrolysierbares Acyloxy oder Dimethylamino substituiertes niederes Alkyl darstellt, mit Ausnahme der (6R,7R)-7-[2-(2-Amino-4-thiazolyl)-2-(methoxyimino)-acetamido]-3-[[(5-carboxy-2-methyl-2H-1,2,4-triazol-3-yl)thio]methyl]-8-oxo-5-thia-1-azabicyclo[4.2.0]oct-2-en-2-carbonsäure,

sowie Säureadditionssalze dieser Ester und Hydrate dieser Ester bzw. Säureadditionssalze.

2. Ester nach Anspruch 1 in der syn-isomeren Form bzw. Gemische, in denen die syn-isomere Form überwiegt.

3. Ester nach Anspruch 1 oder 2, dadurch gekennzeichnet, dass $R^2$ Aethyl, n-Butyl, Carboxymethyl, Hydroxymethyl, Dimethylaminomethyl oder Pivaloyloxymethyl darstellt.

4. Ester nach einem der Ansprüche 1-3, dadurch gekennzeichnet, dass X Schwefel darstellt.

5. Ester nach einem der Ansprüche 1-3, dadurch gekennzeichnet, dass X die Gruppe -SO- darstellt.

6. Ester nach einem der Ansprüche 1-5, dadurch gekennzeichnet, dass Y die Gruppe -CH= darstellt.

7. Ester nach einem der Ansprüche 1-5, dadurch gekennzeichnet, dass Y Stickstoff darstellt.

8. Ester nach einem der Ansprüche 1-7, dadurch gekennzeichnet, dass Z Schwefel darstellt.

9. Ester nach einem der Ansprüche 1-8, dadurch gekennzeichnet, dass $R^1$ Wasserstoff darstellt.

10. Ester nach einem der Ansprüche 1-8, dadurch gekennzeichnet, dass $R^1$ Methyl darstellt.

11. Ester nach einem der Ansprüche 1-8, dadurch gekennzeichnet, dass $R^1$ Carboxymethyl darstellt.

12. Ester nach einem der Ansprüche 1-8, dadurch gekennzeichnet, dass $R^1$ 1-Carboxy-1-methyl-äthyl darstellt.

13. Ester nach einem der Ansprüche 1-12, dadurch gekennzeichnet, dass $R^2$ Methyl darstellt.

14. Dipivaloyloxymethylester gemäss einem der Ansprüche 1-13.

15. Methylen-(5S,6R,7R)-7-[2-(Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]thio]methyl]-8-oxo-5-thia-1-azabicyclo[4.2.0]oct-2-en-2-carboxylat-pivalat-5-oxid sowie Säuradditionssalze dieser Verbindung und Hydrate dieser Verbindung bzw. Säureadditionssalze.

16. Carbonsäuren der allgemeinen Formel

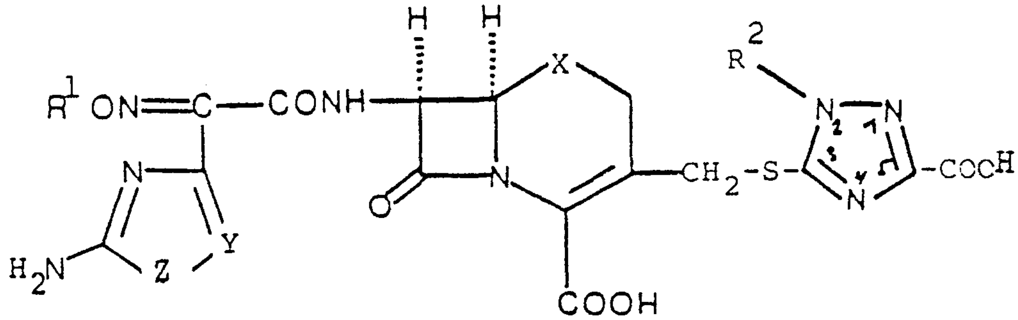

I

in der $R^1$ Wasserstoff, Methyl oder Carboxy-niederes-Alkyl, X Schwefel, Sauerstoff oder eine der Gruppen -SO- und $-SO_2-$, Y die Gruppe -CH= oder Stickstoff, Z Schwefel oder Selen und $R^2$ gegebenenfalls durch Carboxy, Hydroxy, leicht hydrolysierbares Acyloxy oder Dimethylamino substituiertes niederes Alkyl darstellt, mit Ausnahme der (6R,7R)-7-[2-(2-Amino-4-thiazolyl)-2-(methoxyimino)-acetamido]-3-[[(5-carboxy-2-methyl-2H-1,2,4-triazol-3-yl)thio]methyl]-8-oxo-5-thia-1-azabicyclo[4.2.0]oct-2-en-2-carbonsäure,

sowie Salze dieser Verbindungen und Hydrate dieser Verbindungen bzw. Salze.

17. Halbester der allgemeinen Formel

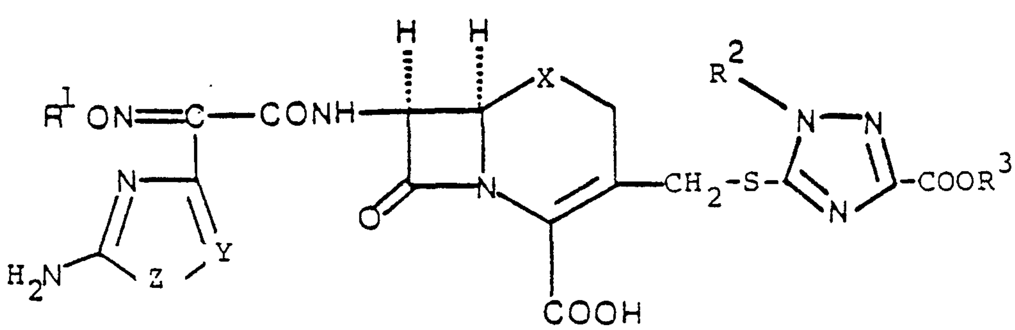

X

worin $R^1$, X, Y, Z und $R^2$ die in Anspruch 1 gegebene Bedeutung haben und $COOR^3$ eine leicht hydrolysierbare Estergruppe darstellt.

18. Verbindungen der allgemeinen Formel

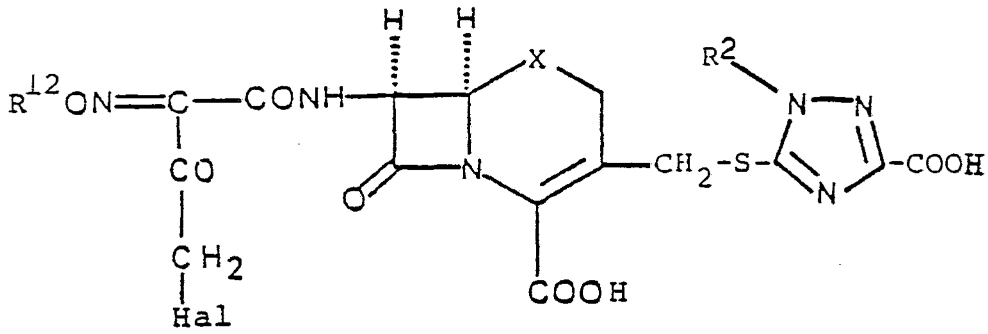

II

in der $R^{12}$ Wasserstoff oder Methyl bedeutet, $R^2$ und X die in Anspruch 1 gegebene Bedeutung haben, Hal ein Halogenatom darstellt und die Carboxygruppe in geschützter Form vorliegen kann,

und leicht hydrolysierbare Ester davon sowie Salze dieser Verbindungen.

19. Verbindungen der allgemeinen Formel

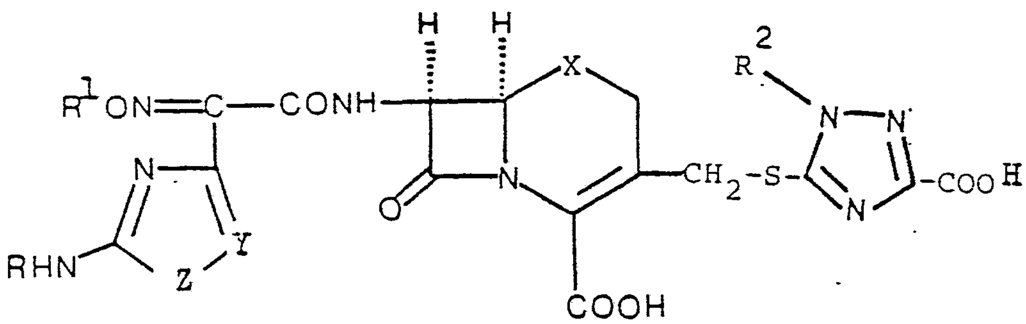

III

in der $R^1$, $R^2$, X, Y und Z die in Anspruch 1 gegebene Bedeutung haben, R eine abspaltbare Schutzgruppe darstellt und die Carboxygruppe in geschützter Form vorliegen kann,

und leicht hydrolysierbare Ester davon sowie Salze dieser Verbindungen.

20. Verbindungen der tautomeren Formeln

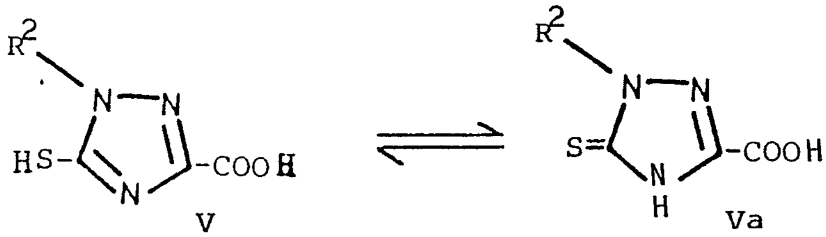

V Va

in der $R^2$ die in Anspruch 1 gegebene Bedeutung hat, jedoch nicht Methyl darstellt,
und leicht hydrolysierbare Ester dieser Verbindungen.

21. Verbindungen der allgemeinen Formel

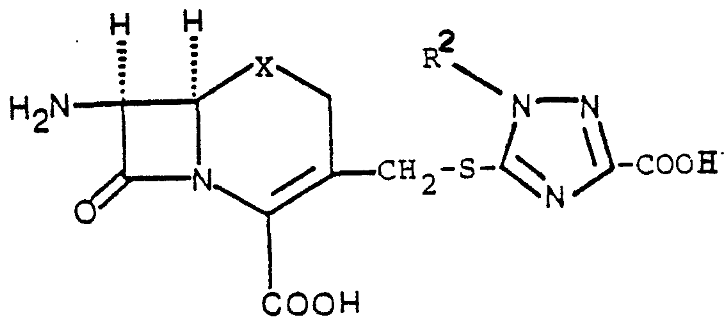

VI

in der X und $R^2$ die in Anspruch 1 gegebene Bedeutung haben und die Carboxygruppe und/oder Aminogruppe in geschützer Form vorliegen kann,
und leicht hydrolysierbare Ester dieser Verbindungen.

22. Verbindungen der allgemeinen Formel

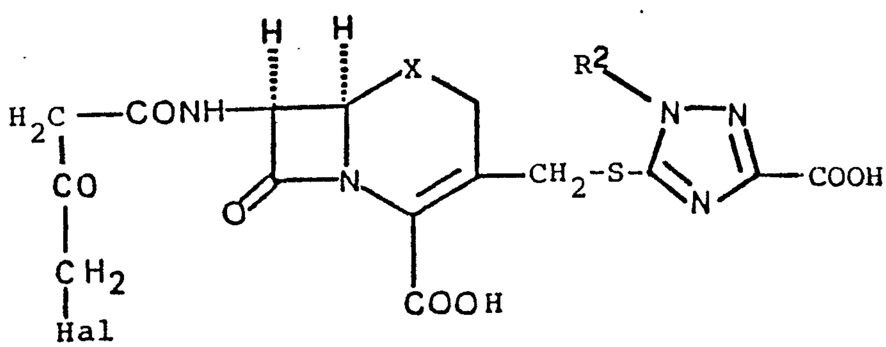

XVI

worin X und $R^2$ die in Anspruch 1 gegebene Bedeutung haben und Hal ein Halogenatom darstellt,
und leicht hydrolysierbare Ester davon.

23. Verbindungen gemäss einem der Ansprüche 1-17 als phamazeutische Wirkstoffe.

24. Verbindungen gemäss einem der Ansprüche 1-15 als pharmazeutische Wirkstoffe zur enteralen, z.B. oralen, Behandlung und Prophylaxe von Infektionskrankheiten.

25. Pharmazeutische Präparate, gekennzeichnet durch einen Gehalt an einer Verbindung gemäss einem der Ansprüche 1-17.

26. Pharmazeutische Präparate zur enteralen, z.B. oralen, Behandlung und Prophylaxe von Infektionskrankheiten gekennzeichnet durch einen Gehalt an einer Verbindung gemäss einem der Ansprüche 1-15.

27. Verfahren zur Herstellung der Verbindungen gemäss einem der Ansprüche 1-15, d.g. dass man die entsprechende Dicarbonsäure, d.h. eine Verbindung der Formel I oder ein Salz dieser Verbindung einer entsprechenden Veresterung unterwirft, erwünschtenfalls ein erhaltenes Produkt S-oxidiert und erwünschtenfalls das erhaltene Produkt in ein Säureadditionssalz oder Hydrat bzw. ein Hydrat dieses Säureadditionssalzes überführt.

28. Verfahren nach Anspruch 27, d.g. dass man als veresterndes Mittel ein Pivaloyloxymethylhalogenid, insbesondere das Jodid, einsetzt.

***

## Patentansprüche für Oesterreich

1. Verfahren zur Herstellung von leicht hydrolysierbaren Estern von Cephalosporinderivaten der allgemeinen Formel

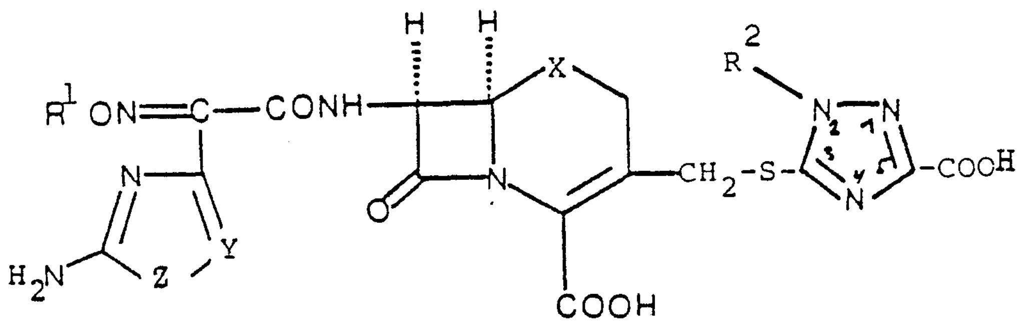

I

in der $R^1$ Wasserstoff, Methyl oder Carboxy-niederes-Alkyl, X Schwefel, Sauerstoff oder eine der Gruppen -SO- und $-SO_2-$, Y die Gruppe -CH= oder Stickstoff, Z Schwefel oder Selen und $R^2$ gegebenenfalls durch Carboxy, Hydroxy, leicht hydrolysierbares Acyloxy oder Dimethylamino substituiertes niederes Alkyl darstellt, mit Ausnahme der (6R,7R)-7-[2-(2-Amino-4-thiazolyl)-2-methoxyimino)-acetamido]-3-[[(5-carboxy-2-methyl-2H-1,2,4-triazol-3-yl)thio]methyl]-8-oxo-5-thia-1-azabicyclo[4.2.0]oct-2-en-2-carbonsäure,

sowie von Säureadditionssalzen dieser Ester und von Hydraten dieser Ester bzw. Säureadditionssalze, dadurch gekennzeichnet, dass man die entsprechende Dicarbonsäure, d.h. eine Verbindung der Formel I oder ein Salz dieser Verbindung einer entsprechenden Veresterung unterwirft, erwünschtenfalls ein erhaltenes Produkt S-oxidiert und erwünschtenfalls das erhaltene Produkt in ein Säureadditionssalz oder Hydrat bzw. ein Hydrat dieses Säureadditionssalzes überführt.

2. Verfahren nach Anspruch 1 zur Herstellung von Estern nach Anspruch 1 in der syn-isomeren Form bzw. von

Gemischen, in denen die syn-isomere Form überwiegt, dadurch gekennzeichnet, dass man eine Ausgangsverbindung mit entsprechender Konfiguration einsetzt oder ein erhaltenes syn/anti-Gemisch auftrennt.

3. Verfahren nach Anspruch 1 oder 2 zur Herstellung von Estern nach Anspruch 1 oder 2, worin $R^2$ Aethyl, n-Butyl, Carboxymethyl, Hydroxymethyl, Dimethylaminomethyl oder Pivaloyloxymethyl darstellt, dadurch gekennzeichnet, dass man entsprechend substituierte Ausgangsverbindungen einsetzt.

4. Verfahren nach einem der Ansprüche 1-3 zur Herstellung von Estern nach einem der Ansprüche 1-3, worin X Schwefel darstellt, dadurch gekennzeichnet, dass man entsprechend substituierte Ausgangsverbindungen einsetzt.

5. Verfahren nach einem der Ansprüche 1-3 zur Herstellung von Estern nach einem der Ansprüche 1-3, worin X die Gruppe -SO- darstellt, dadurch gekennzeichnet, dass man entsprechend substituierte Ausgangsverbindungen einsetzt.

6. Verfahren nach einem der Ansprüche 1-5 zur Herstellung von Estern nach einem der Ansprüche 1-5, worin Y die gruppe -CH= darstellt, dadurch gekennzeichnet, dass man entsprechend substituierte Ausgangsverbindungen einsetzt.

7. Verfahren nach einem der Ansprüche 1-5 zur Herstellung von Estern nach einem der Ansprüche 1-5, worin Y Stickstoff darstellt, dadurch gekennzeichnet, dass man entsprechend substituierte Ausgangsverbindungen einsetzt.

8. Verfahren nach einem der Ansprüche 1-7 zur Herstellung von Estern nach einem der Ansprüche 1-7, worin Z Schwefel darstellt, dadurch gekennzeichnet, dass man entsprechend substituierte Ausgangsverbindungen einsetzt.

9. Verfahren nach einem der Ansprüche 1-8 zur Herstellung von Estern nach einem der Ansprüche 1-8, worin $R^1$ Wasserstoff darstellt, dadurch gekennzeichnet, dass man entsprechend substituierte Ausgangsverbindungen einsetzt.

10. Verfahren nach einem der Ansprüche 1-8 zur Herstellung von Estern nach einem der Ansprüche 1-8, worin $R^1$ Methyl darstellt, dadurch gekennzeichnet, dass man entsprechend substituierte Ausgangsverbindungen einsetzt.

11. Verfahren nach einem der Ansprüche 1-8 zur Herstellung von Estern nach einem der Ansprüche 1-8, worin $R^1$ Carboxymethyl darstellt, dadurch gekennzeichnet, dass man entsprechend substituierte Ausgangsverbindungen einsetzt.

12. Verfahren nach einem der Ansprüche 1-8 zur Herstellung von Estern nach einem der Ansprüche 1-8, worin $R^1$ 1-Carboxy-1-methyl-äthyl darstellt, dadurch gekennzeichnet, dass man entsprechend substituierte Ausgangsverbindungen einsetzt.

13. Verfahren nach einem der Ansprüche 1-12 zur Herstellung von Estern nach einem der Ansprüche 1-12, worin $R^2$ Methyl darstellt, dadurch gekennzeichnet, dass man entsprechend substituierte Ausgangsverbindungen einsetzt.

14. Verfahren nach einem der Ansprüche 1-13 zur Herstellung von Dipivaloyloxymethylestern gemäss einem der Ansprüche 1-13, dadurch gekennzeichnet, dass man als veresterndes Mittel ein Pivaloyloxymethylhalogenid, insbesondere das Jodid, einsetzt.

15. Verfahren nach einem der Ansprüche 1, 2, 5, 6, 8, 10, 13 und 14 zur Herstellung von Methylen-(5S,6R,7R)-7-[2-(Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]thio]methyl]-8-oxo-5-thia-1-azabicyclo[4.2.0]-

oct-2-en-2-carboxylat-pivalat-5-oxid sowie von Säureadditionssalzen dieser Verbindung und von Hydraten dieser Verbindung bzw. Säureadditionssalze, dadurch gekennzeichnet, dass man entsprechend substituierte Ausgangsverbindungen einsetzt.